(19) Europäisches Patentamt
European Patent Office
Office européen des brevets

(11) EP 1 201 238 A1

(12) # EUROPEAN PATENT APPLICATION
published in accordance with Art. 158(3) EPC

(43) Date of publication:
**02.05.2002 Bulletin 2002/18**

(21) Application number: **00948274.6**

(22) Date of filing: **28.07.2000**

(51) Int Cl.⁷: **A61K 31/192**, A61P 29/00, C07C 311/19

(86) International application number:
**PCT/JP00/05057**

(87) International publication number:
**WO 01/08674 (08.02.2001 Gazette 2001/06)**

(84) Designated Contracting States:
**AT BE CH CY DE DK ES FI FR GB GR IE IT LI LU MC NL PT SE**
Designated Extension States:
**AL LT LV MK RO SI**

(30) Priority: **29.07.1999 JP 21496299**

(71) Applicant: **ONO PHARMACEUTICAL CO., LTD.**
**Osaka-shi, Osaka 541-8526 (JP)**

(72) Inventors:
- **MINAMI, Toshiaki**
  **Hirakata-shi, Osaka 573-0031 (JP)**
- **ITO, Seiji**
  **Toyonaka-shi, Osaka 560-0005 (JP)**
- **OHUCHIDA, Shuichi,,**
  **Ono Pharmaceutical Co., Ltd.**
  **Mischima-gun, Osaka 618-8585 (JP)**
- **NAGANAWA, Atsushi,**
  **Ono Pharmaceutical Co., Ltd.**
  **Mishima-gun, Osaka 618-8585 (JP)**
- **MARUYAMA, Takayuki,**
  **Ono Pharmaceutical Co., Ltd.**
  **Mishima-gun, Osaka 618-8585 (JP)**

(74) Representative: **Henkel, Feiler, Hänzel**
**Möhlstrasse 37**
**81675 München (DE)**

(54) **SULFONAMIDE DERIVATIVES AND REMEDIES FOR ALLODYNIA**

(57) An agent for the treatment and/or prevention of allodynia comprising a sulfonamide derivative of formula (W), an ester thereof, a non-toxic salt thereof and a cyclodextrin clathrate thereof as an acrive ingredient and a novel sulfonamide derivative.

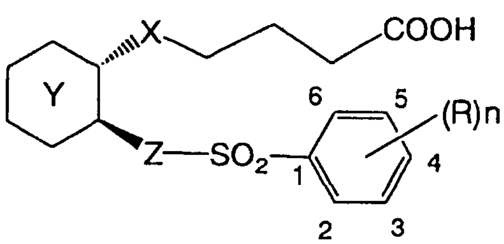

(W)

The compound of formula (W) is useful for the treatment and/or prevention of allodynia caused by peripheral nerve injury (e.g. causalgia, sympathetic reflex dystrophy), central nerve injury (e.g., phantom limb pain, post-zoster neuralgia (post-herpetic pain), thalamic pain, pain after spinal cord injury), and diabetic neuropathy or nerve invasive cancer.

EP 1 201 238 A1

## Description

### Technical Field

The present invention relates to an agent for the treatment and/or prevention of allodynia. More particularly, the present invention relates to an agent for the treatment and/or prevention of allodynia comprising a sulfonamide derivative of formula (W) described hereinafter, an ester thereof, a non-toxic salt thereof or a cyclodextrin clathrate thereof as an active ingredient.

The present invention also relates to a novel sulfonamide derivative of formula (W-A) described hereinafter, an ester thereof, a non-toxic salt thereof or a cyclodextrin clathrate thereof.

### Background

Pain is not only a warning response for the life maintenance against external noxious stimuli and pathological situations in the body but also pain brings harmful stage to the body by itself, that is, pain has binary character.

Under physiological condition, pain is produced as a warning response against a noxious stimulus which activates nociceptor localized in the peripheral tissue followed by the liberation of algogenic substances.

Nociceptor are high-threshold mechanical receptor which transmits pain to central nervous system through thin myelinated Aδ fiber which is primary afferent fiber and polymodal receptor which transmits pain through unmyelinated C fiber. High-threshold mechanical receptor does not react until mechanical stimulus reaches the noxious level. Polymodal receptor reacts to heat and chemical stimulus besides mechanical stimulus. The activity increases depending on the strength of the noxious stimulus.

Under pathological conditions such as inflammation in peripheral tissues and tissue damage, a persistence production of sensitizing and algogenic substances can continuously activate nociceptor, which results in lowering the threshold to respond the noxious stimulus of heat, mechanical and chemical stimuli. This situation brings "hyperalgesia" stage.

Non-noxious stimulus such as tactile stimulation like warm or cold or light touch is transmitted from peripheral non- nociceptor to central nerve system through thick myelinated Aδ fiber, but the stimulus does not cause pain under physiological condition. On the other hand, under pathological condition light touch and cold stimulation can cause pain. This symptom is so called "allodynia". Allodynia is associated with pain caused by peripheral nerve injury (e.g. causalgia, sympathetic reflex dystrophy), central nerve injury (e.g. phantom limb pain, post-zoster neuralgia (post-herpetic pain), thalamic pain, pain after spinal cord injury), diabetic neuropathy or nerve invasive cancer.

The mechanism of induction of allodynia is not sufficiently cleared. Recently prostaglandin (PG), for the first time as a physiologically active substance, could cause allodynia and hyperalgesia by the intrathecal delivery (Pain, 50, 223-9 (1992)). For example, when PGE2 was administered intrathecally, not only hyperalgesia against noxious heat stimulus but also allodynia for non-noxious tactile stimulus can be formed. When PGF2a is administered intrathecally, hyperalgesia is not induced, but allodynia is induced. On the other hand, PGD2 induces hyperalgesia but not induce allodynia. It is also suggested that PGD2 exists as an endogenous producible inhibitory substance for allodynia which is induced by non-noxious tactile stimulus. PGI2 and TXA2 do not induce allodynia or hyperalgesia. As described herein, as to the relationship of PGs and allodynia, though many facts are being revealed but it is not resolved enough.

However, as shown above, it is obvious that 1) normal pain, 2) hyperalgesia and 3) allodynia should be considered to be different. Since each has different mechanisms, it is also obvious that it is necessary to confirm whether the compound is effective on these pains.

As shown above, it is generally known that PGE2 is an algogenic substance with inflammation. It is indicated among PGE2 receptor subtypes, EP1 receptor is related to pain-induction and EP1 antagonist is expected to be useful as an analgesic for normal pains.

It is reported that a compound named ONO-NT-012 has an effect on the suppression of allodynia and that its efficacy is concerned with EP1 receptor (British Journal of Pharmacology, 115, 73-76 (1995)).

On the other hand, the compound described in the claims of EP 312,906 includes the compounds of the present invention of formula (I) and (II) described hereinafter. It is disclosed that the compounds of the specification are useful as TXA2 antagonist; and that they are useful for the prevention and/or treatment of inflammation, hypertension, thrombosis, apoplexy, asthma, myocardial infarction, angina pectoris, brain infarction, acute heart death. But in the specification the compounds of formula (I) and (II) are not specifically described.

The compounds described in the claims of EP 878,465 include the compounds of the present invention of formula (III) and (IV) described hereinafter. It is disclosed that the compounds of the specification are EP1 agonist or antagonist and that they are useful as an antipyretic, analgesic or agent for the treatment of pollakiuria. But in the specification the compounds of formula (III) and (IV) are not specifically described.

In examples 3(d) and 3(a) of EP 608,847, the compounds of formula (V) and (VI) of the present invention are

described. This specification discloses that these compounds bind to PGE2 receptor and show antagonizing or agonizing effect, so that they are useful as PGE2 antagonist or agonist. It discloses PGE2 antagonist has an inhibitory effect of uterine contraction and digestive peristalsis, an analgesic effect and a sleep-inducing effect, and PGE2 agonist has a uterine contractile activity, a promoting effect of digestive peristalsis, a suppressive effect of gastric acid secretion and a hypotensive activity.

In the specification of EP 226,346, the compound of formula (VI) of the present invention is described as compound No. lh-ba (2S-t-5Z). It discloses that the compound inhibits platelet aggregation and vasoconstriction, so it is useful as TXA2 antagonist and has an effect as anti-thrombosis and anti-vasoconstriction agent.

## Disclosure of the invention

The inventors made efforts to screen various compounds in order to find out those having a strong inhibitory effect on allodynia, so that it was found that the compounds of bicyclo structure were useful. As a result of investigation of the compounds for EP1 receptors, the binding affinity was weak and the inhibitory effect on allodynia was not correlated with EP1 antagonist activity. Therefore, it is considered that the expression of anti-allodynia effect of these compounds was not caused by EP1 antagonist effect.

The structures of the compounds were compared on the results obtained from the screening, accordingly, some compounds are effective on allodynia and others are ineffective in spite of similar structure. Therefore it was impossible to ascertain the relationship between structure and anti- allodynia effect. The present inventors have found that some compounds are effective, others are not for allodynia among similar structures. Since the activity was not correlated with the binding activity of various PG receptor and TX receptor generally expected, so it suggested that it was probable that novel unknown target molecule (receptor etc.) are involved.

The present inventors found only some compounds are useful for allodynia, to complete the present invention.

That is, the present invention relates to an agent for the treatment and/or prevention of allodynia comprising, as an active ingredient, one or more compounds selected from sulfonamide derivatives of formula (W):

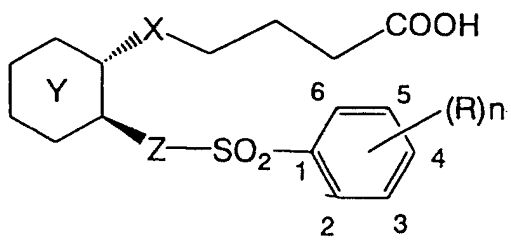

(W)

(wherein each symbol represents a combination of (1), (2) or (3):

(1) ring

Y is

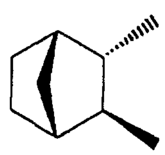

X is

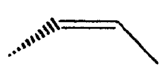

Z is

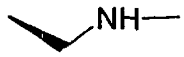

and (R)n is 2-iodo, 2-fluoro or 3,4-difluoro;

(2) ring

Y is

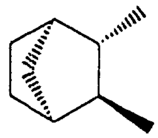

X is

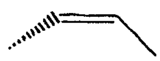

Z is

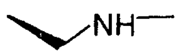

and (R)n is 2,5-difluoro;

(3) ring

Y is

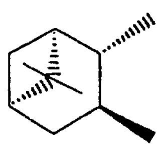

X is

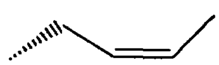

Z is

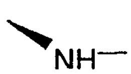

and (R)n is 4-methoxy or hydrogen),

esters thereof, non-toxic salts thereof or cyclodextrin clathrates thereof.

Specific examples of sulfonamide derivatives of above formula (W) are the compounds of formula (I) to (VI)

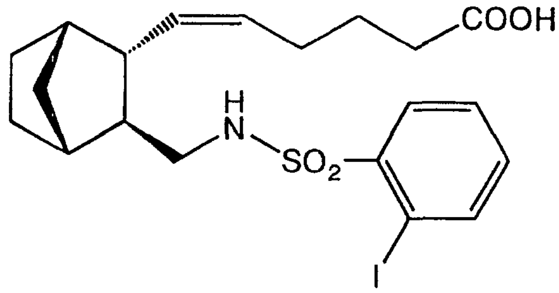

(I)

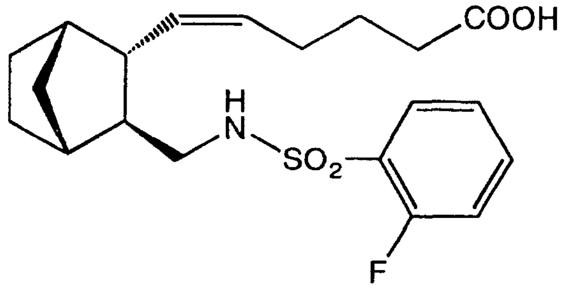

(II)

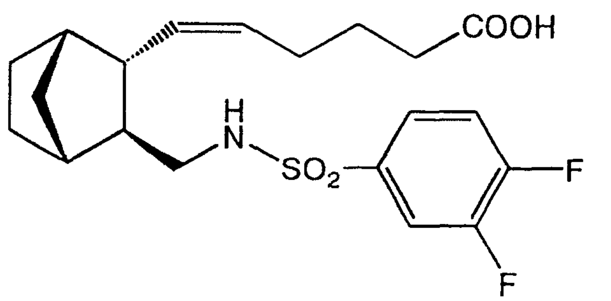

(III)

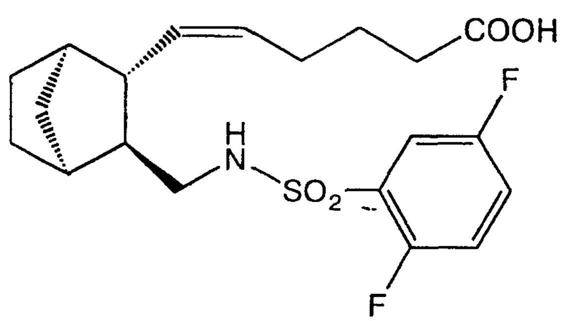

(IV)

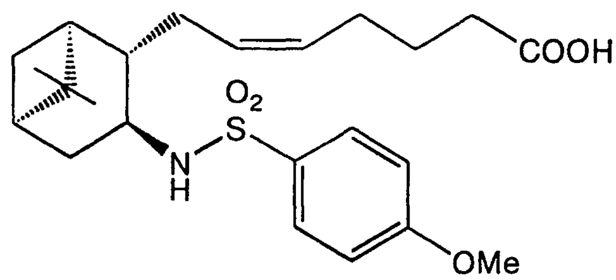

(V)

and

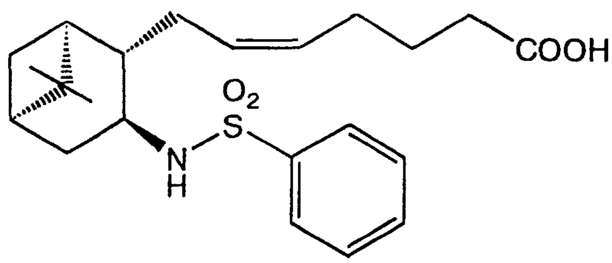

(VI)

.

Hereinafter, "the compounds of the present invention" means a sulfonamide derivative of above formula (W), an ester thereof, a non-toxic salt thereof or a cyclodextrin clathrate thereof.

And sulfonamide derivatives of formula (W-A):

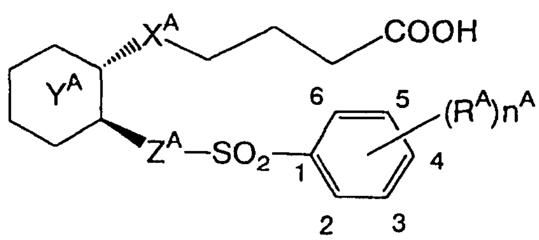

(W-A)

(wherein each symbol represents a combination of (1) or (2):

(1) ring

$Y^A$ is

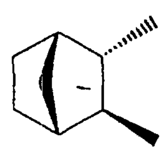

$X^A$ is

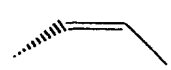

$Z^A$ is

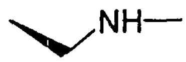

and $(R^A)n^A$ is 2-iodo, 2-fluoro or 3,4-difluoro;

(2) ring

$Y^A$ is

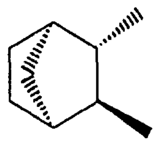

$X^A$ is

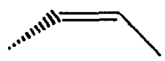

$Z^A$ is

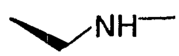

and $(R^A)n^A$ is 2,5-difluoro),

esters thereof, non-toxic salts thereof or cyclodextrin clathrates thereof are not shown specifically though they are included in claim of above laid-open disclosure public patent bulletin . So these compounds are included in the present invention.

**Description**

[Ester]

A sulfonamide derivative of formula (W) may be converted to an ester in a conventional method. Conversion to ester structure improves stability and absorption of the compound. Alkyl esters are preferable, and C1~4 alkyl esters are more preferable.

[Salt]

A sulfonamide derivative of formula (W) may be converted into a corresponding salt in a conventional method. Non-toxic and water-soluble salts are preferable. Appropriate salts includes, a salt of alkali metal (potassium, sodium etc.), a salt of alkaline-earth metal (calcium, magnesium, etc.), an ammonium salt, a pharmaceutically acceptable organic amine (tetramethyl ammonium, triethylamine, methylamine, dimethylamine, cyclopentylamine, benzylamine, phenethylamine, piperidine, monoethanolamine, diethanolamine, tris(hydroxymethyl)amine, lysine, arginine, N-methyl-D-glucamine etc.). The sulfonamide derivative of formula (W), an ester thereof and a non-toxic salt thereof may be converted into a hydrate in a conventional method.

[Clathrates]

A sulfonamide derivative of formula (W), an ester thereof and a non-toxic salt thereof may be converted into cyclodextrin clathrates using α-, β-, γ cyclodextrin or a mixture thereof, by the methods described in the specification

of GB Patent Nos. 1,351,238 and 1,419,221. Conversion into their cyclodextrin clathrates serves to increase the stability and solubility in water of the compounds, and so it is convenient in the use for pharmaceuticals.

[The method for the preparation of the compounds of the present invention]

The compounds of the present invention may be prepared according to the method described in the specifications of EP 312,906, EP 608,847, EP 878,465 or EP 226,346. They may also be prepared according to the method described in the following reference examples and examples.

[Pharmacological activity]

It was confirmed that the compounds of the present invention are useful for the treatment and/or prevention of allodynia and other compounds having similar structure are not effective on the following experiment.

[Pharmacological experiment]

(1) an inhibitory effect on allodynia caused by intrathecal injection of $PGE_2$

Five or six male ddY mice weighing 22± 2 g in a group were used. Under non-anesthesia, the mixture (5µL) of PGE2 (10ng/body) and a compound (10ng/body) with 27-gauge was injected intrathecally with stainless injection needle (0.33 mm, o.d.) between fifth and sixth vertebra lumbar of a mouse, 5. min after administration, to tail from belly side of a mouse was touch-stimulated with brush and allodynia was evaluated by score.

Score is 0: no changed, 1: escaped from touch-stimulus and squeaked, 2: escaped vigorously from touch-stimulus and squeaked loudly. When All the subjects got score 2, it was evaluated as 100% of allodynia expression.

For example, When, in six mice, score 0 is one, score 1 is three and score 2 is two, % of allodynia expression is below:

$$\frac{(0\times1) + (1\times3) + (2\times2)}{6\times2} \times 100 = 58.\ 3(\%)$$

(2) an inhibitory effect on allodynia caused by intrathecal injection of $PGF_{2\alpha}$

The experiment was the same as (1) with the exception that the mixture of $PGF_{2\alpha}$ (1µg/body) and a compound (100ng/body) was used as administered drug and allodynia was evaluated at 15min after administration.

table 1 : % of allodynia expression

| compound | Allodynia caused by $PGE_2$ | Allodynia caused by $PGF_{2\alpha}$ |
|---|---|---|
| formula (I) | 8.3 ± 8.3 | 8.3 ± 8.3 |
| formula (II) | 0.0 ± 0.0 | 8.3 ± 8.3 |
| comparison compound 1 | 100 ± 0.0 | 91.7 ± 8.3 |
| comparison compound 2 | 83.3 ± 10.5 | No test |

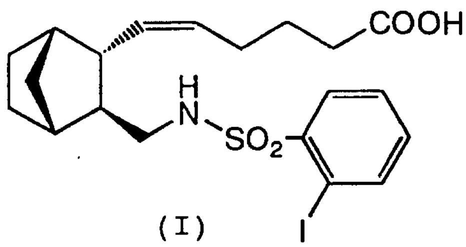

(I)

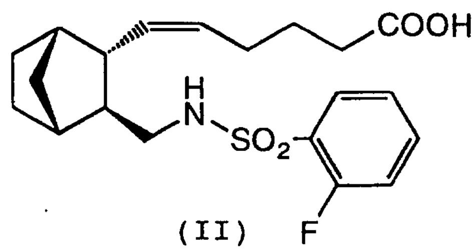

(II)

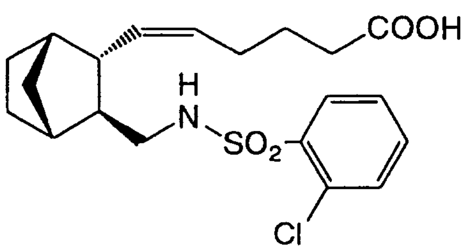

comparison compound 1

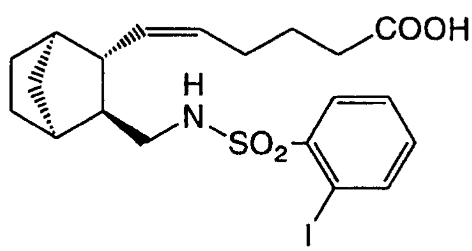

comparison compound 2

Table 2 : % of allodynia expression

| compound | Allodynia caused by $PGE_2$ | Allodynia caused by $PGF_{2\alpha}$ |
|---|---|---|
| Formula(III) | 0.0 ± 0.0 | 8.3 ± 8.3 |
| Formula(IV) | 8.3 ± 8.3 | 0.0 ± 0.0 |
| comparison compound 3 | 91.7 ± 8.3 | No test |
| comparison compound 4 | 75.0 ± 11.2 | No test |

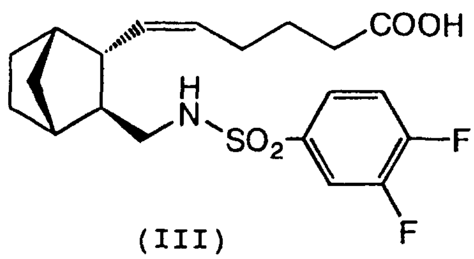

(III)

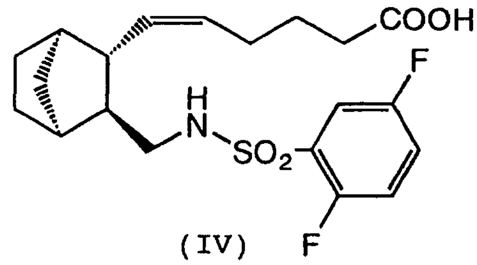

(IV)

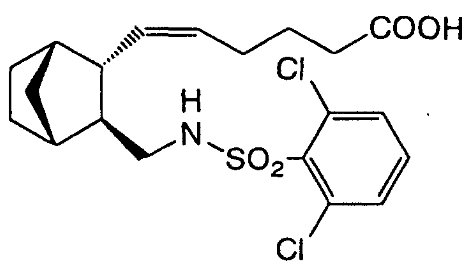

comparison compound 3

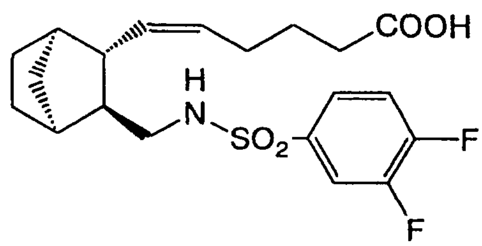

comparison compound 4

table 3 : % of allodynia expression

| compound | Allodynia caused by $PGE_2$ | Allodynia caused by $PGF_{2\alpha}$ |
|---|---|---|
| formula (V) | 8.3 ± 8.3 | 8.3 ± 8.3 |
| formula (VI) | 8.3 ± 8.3 | 0.0 ± 0.0 |
| comparison compound 5 | 83.3 ± 10.5 | 83.3 ± 10.5 |
| comparison compound 6 | 90.0 ± 10.0 | 60.0 ± 10.0 |

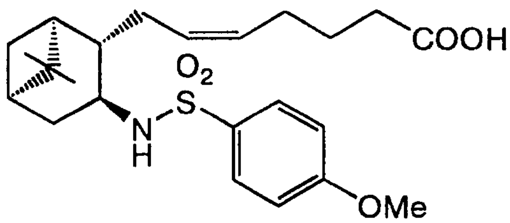

(V)

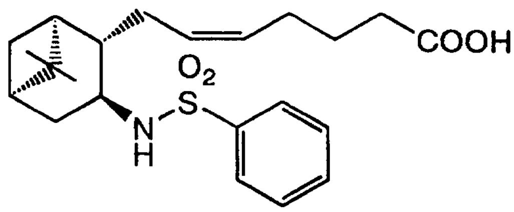

(VI)

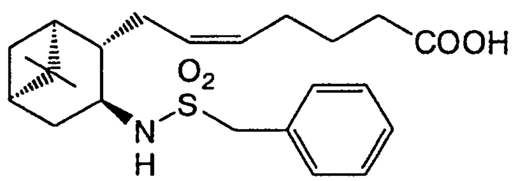

comparison compound 5

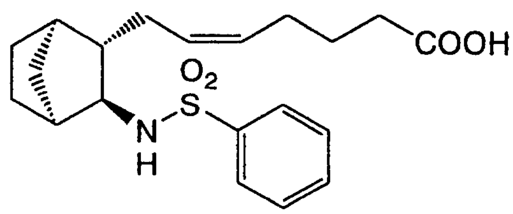

comparison compound 6

As shown in tables 1, 2 and 3, the compounds of the present invention inhibited the allodynia expression potently. On the other hand, the comparison compounds 1 - 6 had no inhibitory activity at all though they have similar structure. Hereinafter, concrete speculation was described.

In table 1, at first glance, it seems that bicyclo[2.2.1]heptane skelton and o-halogen substituted compounds on phenyl part are effective, but the comparison compound 1 which has the same skeleton and having chlorine as o-halogen had no effect. O-iodide substituted phenyl compound having different steric chemistry on bicyclo[2.2.1]heptane skeleton did not have effect.

Table 2 shows that bicyclo[2.2.1]heptane skeleton system compounds having no effect in table 1 are effective if it has two substituents (compound (IV)). But the conversion of substituting position only is ineffective. The compound (III) which converts steric chemistry of bicyclo[2.2.1]heptane skeleton of compound (IV) is effective. The comparative compound 3 which is di-substituted by chlorine though has a different substituting position has no effect. From these results, it is found that it is not easy to expect the activities of comparison compounds and the compounds of the present invention from steric chemistry.

In table 3, bicyclo[3.1.1]heptane skeleton compounds have activity in both cases phenyl has substituents or not (compound (V), (VI)). But when it bears methylene between sulfon and phenyl, they have no effect. The compound combined with bicyclo[2.2.1]heptane skeleton having effect in compound (IV) and unsubstituted phenyl having effect in compound (VI) is ineffective.

From these aspects, it is found that there is no correlationship between ring structure, substituents and anti-allodynia effect. No correlationship between EP1 binding activity and anti-allodynia effect on the comparison compound and the compounds of the present invention was recognized.

[Toxicity]

The toxicity of the compounds of the present invention is very low and therefore the compounds may be considered safe for pharmaceutical use. For example, the maximal tolerated dose with oral administration of the compounds of formula (I), (IV) and (VI) was more than 2000 mg/kg in rat.

[Application to Pharmaceutical Compositions]

The compound of the present invention is useful for the treatment and/or prevention of allodynia. For example, it is useful for the prevention and/or the treatment of allodynia caused by peripheral nerve injury (e.g. causalgia, sympathetic reflex dystrophy), central nerve injury (e.g., phantom limb pain, post-zoster neuralgia (post-herpetic pain), thalamic pain, pain after spinal cord injury), and diabetic neuropathy or nerve invasive cancer.

For the purpose above described, the compounds of formulae (W), non-toxic salts thereof, esters thereof and cyclodextrin clathrates thereof may be normally administered systemically or locally, usually by oral or parenteral administration. Converting to non-toxic salts, esters or cyclodextrin clathrates eliminates irritation, and improves absorption and stability.

The doses to be administered are determined depending upon age, body weight, symptom, the desired therapeutic effect, the route of administration, and the duration of the treatment etc. In the human adult, the doses per person per dose are generally from 1 μg to 100 mg, by oral administration, from once up to several times per day, and from 0.1 μg to 10 mg, by parenteral administration (preferably intravenously) from once up to several times per day, or by continuous administration for from 1 hour to 24 hours per day into vein.

As mentioned hereinbefore, the doses to be administered depend upon various conditions. Therefore, there are cases in which doses lowerthan or greater than the ranges specified hereinbefore may be used.

The compounds of the present invention may be administered in the form, for example, of solid compositions, liquid compositions or other compositions for oral administration, or injections, liniments or suppositories etc. for parenteral administration.

Solid compositions for oral administration include compressed tablets, pills, capsules, dispersible powders, and granules etc.

Capsules include hard capsules and soft capsules.

In these solid compositions, one or more of the active compound(s) are admixed with at least one inert diluent, e.g. lactose, mannitol, mannit, glucose, hydroxypropyl cellulose, microcrystalline cellulose, starch, polyvinyl pyrrolidone, magnesium metasilicate aluminate. The composition may comprise, according to the conventional manner, additives other than inert diluents, e.g. lubricating agents such as magnesium stearate, disintegrating agents such as cellulose calcium glycolate, agents to assist dissolution such as glutamic acid, aspartic acid. The tablets or pills may, if desired, be coated with film of gastric or enteric material such as sugar, gelatin, hydroxypropyl cellulose, hydroxypropyl cellulose phthalate etc. or be coated with more than one film. Coating may include containment within capsules of absorbable materials such as gelatin.

Liquid compositions for oral administration include pharmaceutically acceptable emulsions, solutions, syrups and elixirs etc. In such liquid compositions, one or more of the active compound(s) may be contained in inert diluent (s) commonly used in the art (e.g. purified water, ethanol). Besides inert diluents, such compositions may also comprise assisting agents (e.g. wetting agents, suspending agents), sweetening agents, flavoring agents, perfuming agents and preserving agents.

Other compositions for oral administration include spray compositions which comprise one or more of the active compound(s), prepared by methods known *per se*. Spray compositions may comprise stabilizing agents such as sodium sulfite hydride, isotonic buffers such as sodium chloride, sodium citrate or citric acid. For the preparation of such spray compositions, for example, the method described in the United States Patent No. 2,868,691 or No. 3,095,355 may be used.

Injections for parenteral administration in present invention include sterile aqueous or non-aqueous solutions, suspensions and emulsions. Aqueous solutions and suspensions include, for example, distilled water for injection and physiological salt solution. Non-aqueous solution and suspensions include, for example, propylene glycol, polyethylene glycol, vegetable oil such as olive oil, alcohol such as ethanol, POLYSORBATE80 (registered trademark) etc. These compositions may comprise assisting agents such as preserving agents, wetting agents, emulsifying agents, dispersing agents, stabilizing agents, agents assisting dissolution (e.g. glutamic acid, aspartic acid etc.). They may be sterilized for example, by filtration through a bacteria-retaining filter, by incorporation of sterilizing agents in the compositions or by irradiation. They may also be manufactured in the form of sterile solid compositions which may be dissolved in sterile water or some other sterile solvent(s) for injection before use.

Other compositions for parenteral administration include liquids for external use, and ointment, endermic liniments, suppositories for rectal administration and pessaries for vaginal administration etc. which comprise one or

more of active compound(s) and may be prepared by conventional methods:

**The best mode for carrying out the invention**

The following reference examples and examples are intended to illustrate the present invention, but do not limit them.
In chromatographic separations and TLC, the solvents in parenthesis show the eluting and developing solvents and the ratios of the solvents used are by volume.
The solvents in parenthesis in NMR show the solvents used for measurement.

Reference example 1

sodium o-iodobenzenesulfonic acid

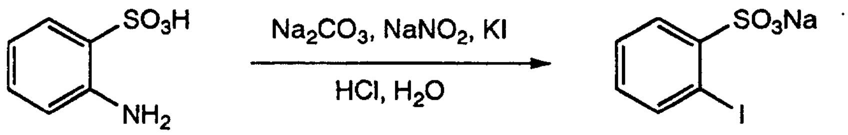


A suspension of o-aminobenzenesulfonic acid (10 g) in water (65 ml) was stirred at the room temperature and added thereto sodium carbonate (3.06 g). To the reaction mixture was added sodium nitrite (4.2 g) little by little. The mixture was stirred at 0°C for 20 minutes. The reaction mixture was poured into a mixture of concentrated hydrochloric acid (10 ml) and ice (50 g) and stirred. To the mixture was added an aqueous solution (10 ml) of potassium iodide (10.5 g) slowly. The reaction solution was stirred at the temperature of from 0 to 100°C for 1.5 hours and concentrated to remove water. The residue was collected to give the title compound (9.78 g) as a yellow sepia powder.
NMR (DMSO-$d_6$) : δ 7.93 (dd, J=8.0, 1.5 Hz, 1H), 7.88 (dd, J=7.5, 1.5 Hz, 1H), 7.34 (dt, J=8.0, 1.5 Hz, 1H), 7.00 (dt, J=7.5, 1.5 Hz, 1H).

Reference example 2

o-iodobenzenesulfonyl chloride

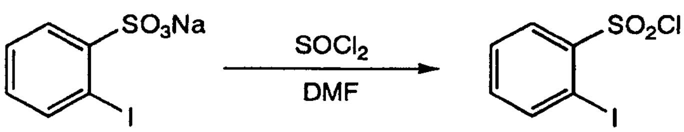


Under atmosphere of argon, a solution of the sodium salt prepared in reference example 1 (5 g) in DMF (30 ml) was stirred. To the solution was added thionyl chloride (2.6 ml) dropwise and the mixture was stirred at 0°C for 1 hour. After the reaction was terminated, to the mixture was added water. The mixture was extracted with ethyl acetate three times. The organic layer was washed with water twice and with brine once. The mixture was dried over anhydrous magnesium sulfate, concentrated and purified by column chromatography on silica gel (ethyl acetate : hexane = 1 : 5) to give the title compound (3.41 g) as a beige solid.
NMR ($CDCl_3$) : δ 8.25 (dd, J=8.0, 1.5 Hz, 1H), 8.21 (dd, J=8.0, 1.0 Hz, 1H), 7.59 (dt, J=8.0, 1.0 Hz, 1H), 7.35 (dt, J=8.0, 1.5 Hz, 1H);
TLC : Rf 0.33 (ethyl acetate : hexane = 1 : 10).

Example 1

methyl 6-[(1S,2S,3S,4R)-3-(2-iodophenylsulfonylaminomethyl) bicyclo[2.2.1] heptan-2-yl]-5Z-hexenate

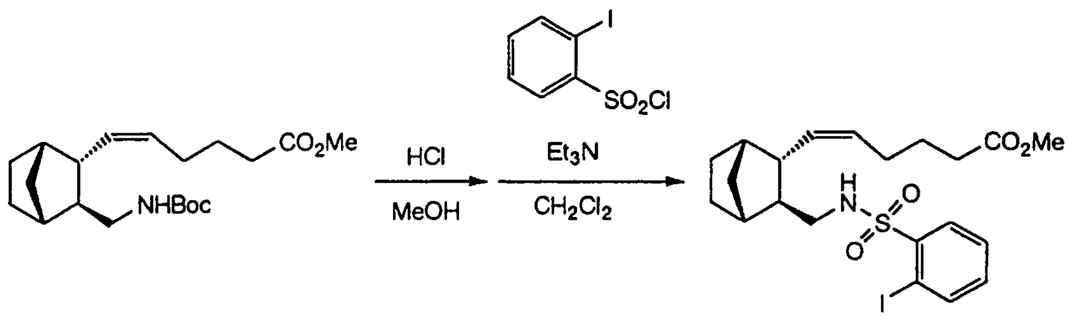


A solution of methyl 6-[(1S,2S,3S,4R)-3-(t-butoxycarbonylaminomethyl) bicyclo[2.2.1]heptan-2-yl]-5Z-hexenate (100 mg) in methanol (2 ml) was stirred at room temperature. To the mixture was added 4N hydrochloric acid-dioxane solution (1 ml) and the mixture was stirred at room temperature for 16 hours. After the reaction was terminated, the mixture was concentrated to give amine hydrochloride.

Under the atmosphere of argon, the amine hydrochloride was dissolved in methylene chloride (3 ml). To the solution was added triethylamine (0.1 ml). 2-iodophenylsulfonyl chloride (112 mg) was added thereto with stirring at 0 °C and the mixture stirred at 0°C for 1 hour. After the reaction was terminated, water was added thereto and the mixture was extracted with ethyl acetate three times. The organic layer was washed with water and 1N hydrochloric acid twice and with brine once. The mixture was dried over anhydrous magnesium sulfate and purified by column chromatography on silica gel (ethyl acetate : hexane = 1 : 5 -> 1 : 1) to give the title compound (139 mg) as a colorless oil.

NMR ($CDCl_3$) : δ 8.16 (dd, J=8.0, 1.5 Hz, 1H), 8.07 (dd, J=8.0, 1.0 Hz, 1H), 7.51 (dt, J=8.0, 1.0 Hz, 1H), 7.21 (dt, J=8.0, 1.5 Hz, 1H), 5.45-5.15 (m, 3H), 3.66 (s, 3H), 2.69 (t, J=6.5 Hz, 2H), 2.30 (t, J=7.5 Hz, 2H), 2.20 (m, 1H), 2.15-2.00 (m, 4H), 1.80-1.00 (m, 9H);

TLC : Rf 0.46 (ethyl acetate : hexane =1 : 3).

Example 2

6-[(1S,2S,3S,4R)-3-(2-iodophenylsulfonylaminomethyl)bicyclo[2.2.1]heptan-2-yl]-5Z-hexenoic acid (represented by formula (I))

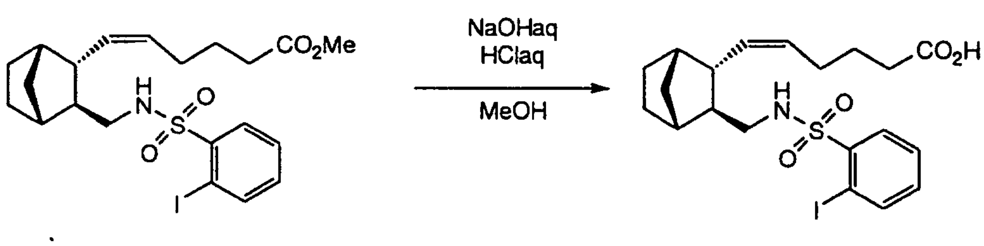


(I)

The methyl ester prepared in example 1 (139 mg) was dissolved in methanol (3 ml). The solution was added 2N aqueous solution of sodium hydroxide (1 ml) with stirring at room temperature. The mixture was stirred for 3 hours. After the reaction was terminated, to the mixture was added 1 N hydrochloric acid and the reaction solution was extracted with ethyl acetate three times. The organic layer was washed with water and 1 N hydrochloric acid twice and with brine once, dried over anhydrous magnesium sulfate, concentrated to give the title compound (128 mg) as a

colorless oil.
NMR (200MHz, $CDCl_3$) : δ 8.16 (dd, J=8.0, 1.5Hz, 1H), 8.07 (dd, J=8.0, 1.0Hz, 1H), 7.51 (dt, J=8.0, 1.0Hz, 1H), 7.22 (dt, J=8.0, 1.5Hz, 1H), 5.45-5.20 (m, 3H), 2.69 (t, J=6.5Hz, 2H), 2.36 (t, J=7.5Hz, 2H), 2.30-1.90 (m, 5H), 1.80-1.00 (m, 9H);
TLC : Rf 0.53 (ethyl acetate : hexane : acetic acid =7 : 12 : 1).

Example 2(a) ~ 2(e)

By the same procedures as described in reference example 1, 2 and 3, and example 1 and 2, the following compounds are obtained.

Example 2(a)

6-[(1S,2S,3S,4R)-3-(2-fluorophenylsulfonylaminomethyl) bicyclo[2.2.1]heptan-2-yl]-5Z-hexenoic acid (represented by formula (II))

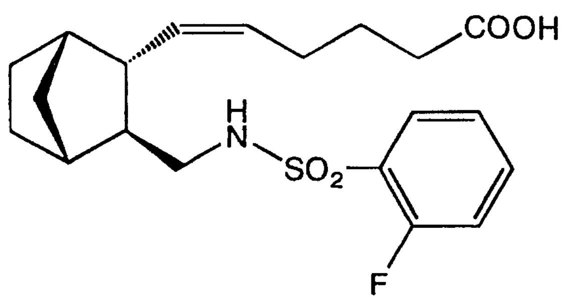

(II)

NMR (300MHz, $CDCl_3$) : δ 7.89 (1H, dt, J=1.5, 7.8Hz), 7.62-7.52 (1H, m), 7.34-7.16 (2H, m), 5.44-5.28 (2H, m), 4.91 (1H, t, J=5.7Hz), 2.93-2.70 (2H, m), 2.36 (2H, t, J=7.8Hz), 2.30-1.88 (6H, m), 1.82-1.64 (2H, m), 1.62-1.42 (2H, m), 1.42-1.02 (4H, m);
TLC : Rf 0.52 (chloroform : methanol = 9 : 1).

Example 2(b)

6-[(1S,2S,3S,4R)-3-(3,4-difluorophenylsulfonylaminomethyl) bicyclo[2.2.1] heptan-2-yl]-5Z-hexenoic acid (represented by formula (III))

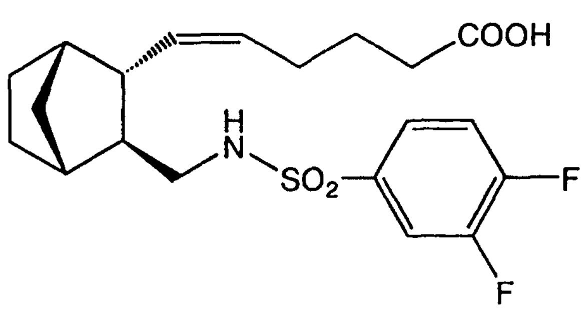

(III)

NMR (300MHz, $CDCl_3$) : δ 7.74-7.60 (m, 2H), 7.37-7.27 (m, 1H), 5.45-5.29 (m, 2H), 5.07 (t, J=6.3 Hz, 1H), 3.00-2.88 (m, 1H), 2.74-2.64 (m, 1H), 2.50-2.20 (m, 4H), 2.14-1.06 (m, 12H);
TLC : Rf 0.31 (hexane : ethyl acetate = 2 : 1).

Example 2(c)

6-[(1R,2S,3S,4S)-3-(2,5-difluorophenylsulfonylaminomethyl) bicyclo[2.2.1] heptan-2-yl]-5Z-hexenoic acid (represented by formula (IV))

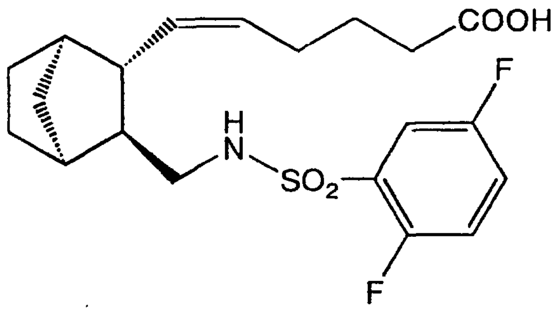

(IV)

NMR (200MHz, $CDCl_3$) : δ 7.62 (m, 1H), 7.22 (m, 2H), 5.22 (m, 2H), 4.83 (m, 1H), 3.04 (m, 2H), 2.35 (t, J=7.6 Hz, 2H), 2.20 (m, 1H), 2.10 (m, 2H), 1.89 (m, 1H), 1.0-1.8 (m, 10H);
TLC : Rf 0.39 (chloroform : methanol = 9 : 1).

Example 2(d)

7-[(1S,2S,3S,5R)-3-(4-methoxybenzenesulfonylamino)-6,6-dimethylbicyclo [3.1.1]heptan-2-yl]-5Z-heptenoic acid (represented by formula (V))

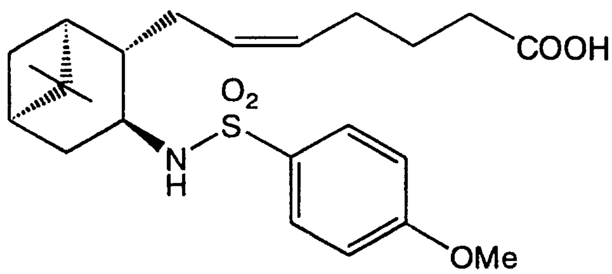

(V)

NMR ($CDCl_3$) : δ 7.82 (2H, dt), 6.96 (2H, dt), 5.38 (2H, m), 5.00 (1H, br), 3.85 (3H, s), 3.58 (1H, m), 2.40-1.40 (14H, m), 1.16 (3H, s), 0.93 (3H, s), 0.76 (1H, d);
TLC : Rf 0.50 (methanol : methylene chloride = 1 : 10).

Example 2(e)

7-[(1S,2S,3S,5R)-3-benzenesulfonylamino-6,6-dimethylbicyclo[3.1.1]heptan-2-yl]-5Z-heptenoic acid (represented by formula (VI))

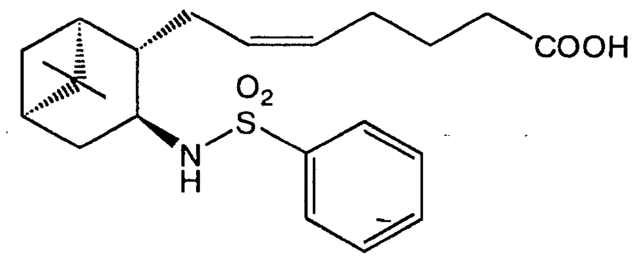

(VI)

NMR ($CDCl_3$) : δ 7.92 (2H, m), 7.55 (3H, m), 5.34 (3H, m), 3.66 (1H, m), 2.40-1.44 (14H, m), 1.16 (3H, s), 0.94 (3H, s), 0.79 (1H, d);
TLC : Rf 0.50 (methanol: methylene chloride = 1: 10).

Synthesis of the comparison compounds

By the same procedures as described in reference example 1,2, and 3, and example 1 and 2, the following compounds are obtained.

Comparison compound 1

6-[(1S,2S,3S,4R)-3-(2-chlorophenylsulfonylaminomethyl) bicyclo[2.2.1] heptan-2-yl]-5Z-hexenoic acid

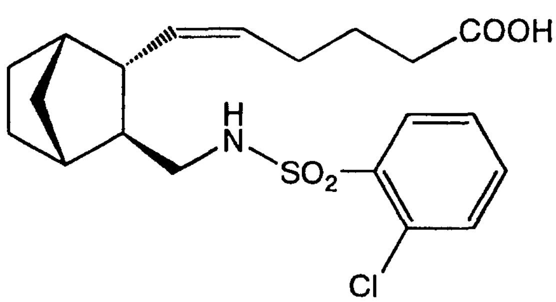

NMR (200MHz, $CDCl_3$) : δ 8.12-8.04 (1H, m), 7.56-7.49 (2H, m), 7.49-7.37 (1H, m), 5.50-5.30 (2H, m), 5.09 (1H, t, J=6.0Hz), 2.78-2.68 (2H, m), 2.35 (2H, t, J=7.4Hz), 2.30-1.95 (5H, m), 1.85-1.00 (9H, m);
TLC : Rf 0.27 (n-hexane : ethyl acetate : acetic acid =3 : 1 : 0.04) .

Comparison compound 2

6-[(1R,2S,3S,4S)-3-(2-iodophenylsulfonylaminomethyl) bicyclo[2.2.1]heptan-2-yl]-5Z-hexenoic acid

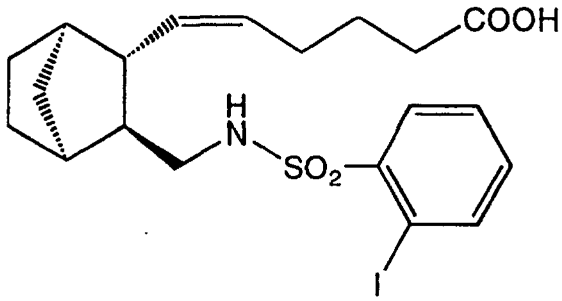

NMR (200MHz, $CDCl_3$) : δ 8.13 (dd, J=7.6, 1.6 Hz, 1H), 8.07 (m, 1H), 7.52 (m, 1H), 5.22 (m, 2H), 5.07 (m, 1H), 7.20 (dd, J=7.6, 1.6 Hz, 1H), 5.22 (m, 2H), 5.09 (m, 1H), 2.98 (m, 1H), 2.78 (m, 1H), 2.34 (t, J=7.2 Hz, 2H), 2.18 (m, 1H), 2.10 (m, 2H), 1.88 (m, 1H), 1.65 (m, 4H), 1.43 (m, 2H), 1.08-1.30 (m, 6H);
TLC : Rf 0.44 (chloroform : methanol =9:1)

Comparison compound 3

6-[(1S,2S,3S,4R)-3-(2,6-dichlorophenylsulfonylaminomethyl) bicyclo[2.2.1] heptan-2-yl]-5Z-hexenoic acid

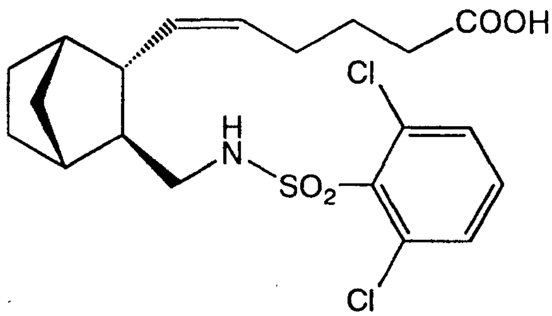

NMR (300MHz, $CDCl_3$) : δ 7.50-7.45 (m, 2H), 7.38-7.32 (m, 1H), 5.47-5.28 (m, 3H), 2.94-2.77 (m, 2H), 2.37 (t, J=7.5 Hz, 2H), 2.30-2.20 (m, 1H), 2.20-2.04 (m, 3H), 2.03-1.97 (m, 1H), 1.80-1.64 (m, 2H), 1.60-1.44 (m, 2H), 1.44-1.00 (m, 5H);
TLC : Rf 0.36 (chloroform : methanol = 9: 1)

Comparison compound 4

6-[(1R,2S,3S,4S)-3-(3,4-difluorophenylsulfonylaminomethyl) bicyclo[2.2.1] heptan-2-yl]-5Z-hexenoic acid

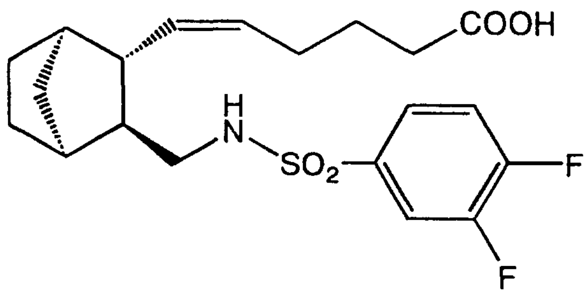

NMR (200MHz, $CDCl_3$) : δ 7.70 (m, 2H), 7.33 (m, 1H), 5.22 (m, 2H), 4.74 (m, 1H), 2.94 (m, 2H), 2.37 (t, J=7.8 Hz, 2H), 2.17 (m, 1H), 2.10 (m, 2H), 1.89 (m, 1H), 1.0-1.8 (m, 10H);
TLC : Rf 0.41 (chloroform : methanol =9 : 1)

Comparison compound 5

7-[(1S,2S,3S,5R)-3-benzylsulfonylamino-6,6-dimethylbicyclo[3.1.1]heptan-2-yl]-5Z-heptenoic acid

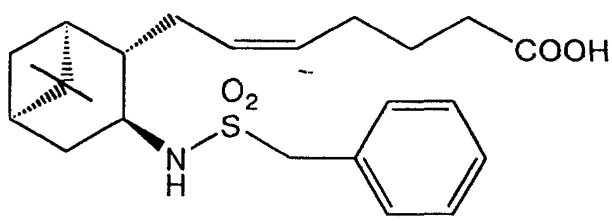

NMR ($CDCl_3$) : δ 7.40 (5H, m), 5.42 (2H, m), 4.86 (1H, d), 4.28 (2H, d), 3.74 (1H, br), 1.22 (3H, s), 1.00 (3H, s), 0.80 (1H, d);
TLC : Rf 0.50 (methanol: methylene chloride = 1 : 10).

Comparison compound 6

6-[(1 R,2S,3S,4S)-3-phenylsulfonylaminobicyclo[2.2.1]heptan-2-yl]-5Z-heptenoic acid

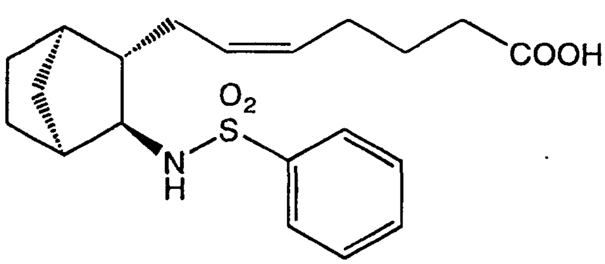

NMR ($CDCl_3$) : δ 7.81-7.91 (2H, m), 7.42-7.61 (3H, m), 5.29 (2H, m), 2.99 (1H, m), 2.34 (2H, t), 0.82-2.22 (17H, m).

Formulation example 1

The following components were admixed in a conventional technique, punched out to give 100 tablets each containing 5 mg of active ingredient.

| | |
|---|---|
| 6-[(1S,2S,3S,4R)-3-(2-iodophenylsulfonylaminomethyl) bicyclo[2.2.1]heptan2-yl]-5Z-hexenoic acid | 500 mg |
| calcium carboxymethylcellulose | 200 mg |
| magnesium stearate | 100 mg |
| microcrystalline cellulose | 9.2 g |

Formulation example 2

The following components were admixed in a conventional technique. The solution was sterilized in a conventional technique, filled in ampoules 10 ml each and freeze-dried in a conventional technique to give 100 ampoules each containing 2 mg of active ingredient.

| | |
|---|---|
| 6-[(1S,2S,3S,4R)-3-(2-iodophenylsulfonylaminomethyl) bicyclo[2.2.1]heptan2-yl]-5Z-hexenoic acid | 200 mg |
| mannite | 50 g |
| distilled water | 1000 ml |

## Claims

**1.** An agent for the treatment and/or prevention of allodynia comprising, as an active ingredient, one or more compounds selected from sulfonamide derivatives of formula (W):

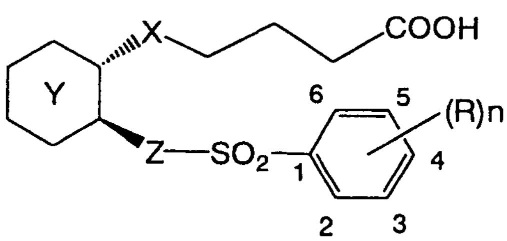

(W)

(wherein each symbol represents a combination of (1), (2) or (3):

(1) ring

Y is

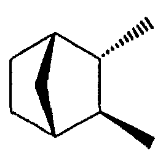

X is

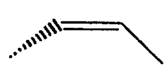

Z is

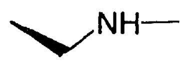

and (R)n is 2-iodo, 2-fluoro or 3,4-difluoro;

(2) ring

Y is

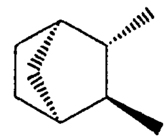

X is

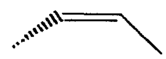

Z is

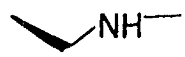

and (R)n is 2,5-difluoro;

(3) ring

Y is

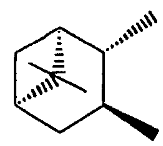

X is

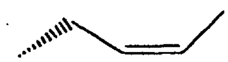

Z is

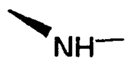

and (R)n is 4-methoxy or hydrogen), esters thereof, non-toxic salts thereof or cyclodextrin clathrates thereof.

2. An agent for the treatment and/or prevention according to claim 1, wherein pain is allodynia caused by peripheral nerve injury, central nerve injury, diabetic neuropathy or nerve invasive cancer.

3. A sulfonamide derivative of formula (W-A):

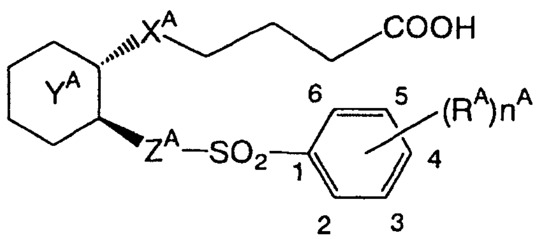

(W-A)

(wherein each symbol represents a combination of (1) or (2):

(1) ring

$Y^A$ is

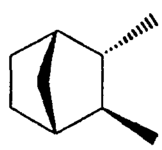

$X^A$ is

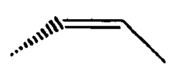

$Z^A$ is

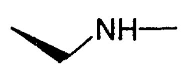

and $(R^A)n^A$ is 2-iodo, 2-fluoro or 3,4-difluoro;

(2) ring

$Y^A$ is

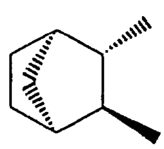

$X^A$ is

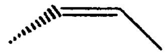

$Z^A$ is

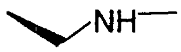

and $(R^A)n^A$ is 2,5-difluoro), an ester thereof, a non-toxic salt thereof or a cyclodextrin clathrate thereof.

## INTERNATIONAL SEARCH REPORT

International application No. PCT/JP00/05057

A. CLASSIFICATION OF SUBJECT MATTER
Int.Cl[7] A61K31/192, A61P29/00, C07C311/19

According to International Patent Classification (IPC) or to both national classification and IPC

B. FIELDS SEARCHED

Minimum documentation searched (classification system followed by classification symbols)
Int.Cl[7] A61K31/192, A61P29/00, C07c311/19

Documentation searched other than minimum documentation to the extent that such documents are included in the fields searched
REGISTRY(STN), CAPLUS(STN)

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | EP, 608847, A1 (Ono Pharmaceutical Co., Ltd.), 03 August, 1994 (03.08.94), (Claims, page 39), & JP, 6-279395, A & CA, 2113787, A & US, 5663417, A | 1-3 |
| A | MINAMI Toshiaki et al., "Characterization of EP-receptor subtypes involved in allodynia and hyperalgesia induced by intrathecal administration of prostaglandin E2 to mice", Br. J. Pharmacol., Vol. 112, no.3 (1994) pp.735-740 | 1-3 |
| A | EP, 878465, A2 (Ono Pharmaceutical Co., Ltd.), 18 November, 1998 (18.11.98), (Claims, page 9), & CA, 2237709, A & AU, 986608, A & JP, 11-29548, A | 1-3 |
| A | WO, 97/00853, A1 (Shionogi & Co., Ltd.,), 09 January, 1997 (09.01.97), (Claims, pages 95-96), & CA, 2225250, A & AU, 9661370, A & EP, 837052, A1 | 1-3 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 October, 2000 (20.10.00) | 31 October, 2000 (31.10.00) |
| Name and mailing address of the ISA/ Japanese Patent Office | Authorized officer |
| Facsimile No. | Telephone No. |

Form PCT/ISA/210 (second sheet) (July 1992)